# EUROPÄISCHE PATENTANMELDUNG

(11) **EP 2 193 813 A2**
(43) Veröffentlichungstag der Anmeldung: **09.06.2010**
(21) Anmeldenummer: 09175459.8
(22) Anmeldetag: 10.11.2009
(51) Int. Cl.: A61L 31/10, A61L 31/14, A61L 31/16

(54) **Wirkstoffbeschichtetes Medizinprodukt, Verfahren zu dessen Herstellung und dessen Verwendungen**

(30) Priorität: 03.12.2008 DE 102008044316
(71) Anmelder: Biotronik VI Patent AG, 6341 Baar (CH)
(72) Erfinder: Wittchow, Eric, 90403, Nürnberg (DE)
(74) Vertreter: Lindner-Vogt, Karin L.

(57) **Zusammenfassung**

Die vorliegende Erfindung betrifft ein Medizinprodukt implantierbar oder einführbar in ein Gefäßsystem eines menschlichen oder tierischen Organismus mit einer erfindungsgemäßen Wirkstoffbeschichtung auf der Oberfläche des Medizinproduktes, ein Verfahren zur Herstellung eines in ein Gefäßsystem eines menschlichen oder tierischen Organismus implantierbaren oder einführbaren Medizinproduktes mit einer erfindungsgemäßen Wirkstoffbeschichtung, eine erfindungsgemäße Wirkstoffbeschichtung für ein in ein Gefäßsystem eines menschlichen oder tierischen Organismus implantierbares oder einführbares Medizinprodukt, Verwendungen der erfindungsgemäßen Wirkstoffbeschichtung zur Herstellung eines in ein Gefäßsystem eines menschlichen oder tierischen Organismus implantierbaren oder einführbaren Medizinproduktes sowie ein Verfahren zur Behandlung einer Stenose etc. im Gefäßsystem eines menschlichen oder tierischen Organismus.

## Beschreibung

### Technisches Gebiet:

Die vorliegende Erfindung betrifft ein Medizinprodukt implantierbar oder einführbar in ein Gefäßsystem eines menschlichen oder tierischen Organismus mit einer erfindungsgemäßen Wirkstoffbeschichtung auf der Oberfläche des Medizinproduktes, ein Verfahren zur Herstellung eines in ein Gefäßsystem eines menschlichen oder tierischen Organismus implantierbaren oder einführbaren Medizinproduktes mit einer erfindungsgemäßen Wirkstoffbeschichtung, eine erfindungsgemäße Wirkstoffbeschichtung für ein in ein Gefäßsystem eines menschlichen oder tierischen Organismus implantierbares oder einführbares Medizinprodukt, Verwendungen der erfindungsgemäßen Wirkstoffbeschichtung zur Herstellung eines in ein Gefäßsystem eines menschlichen oder tierischen Organismus implantierbaren oder einführbaren Medizinproduktes sowie ein Verfahren zur Behandlung einer Stenose etc. im Gefäßsystem eines menschlichen oder tierischen Organismus.

### Hintergrund der Erfindung:

Im Allgemeinen werden ins Gefäßsystem implantierbare oder einführbare Medizinprodukte, wie beispielsweise Stents oder Angioplastie-Ballons, zur Behandlung von Stenosen insbesondere mittels des Angioplastie-Ballon-Verfahrens verwendet.

Stents im Allgemeinen sind endovaskuläre (periphere oder koronare) Prothesen bzw. Implantate, die beispielsweise zur Behandlung von Stenosen, aber auch zur Behandlung von Aneurysmen bekannt sind.

Stents weisen grundsätzlich eine Tragstruktur auf, die geeignet ist, die Wand eines Gefäßes in geeigneter Weise abzustützen, um so das Gefäß zu weiten bzw. ein Aneurysma zu überbrücken. Stents werden dazu in einem komprimierten Zustand in das Gefäß eingeführt, dann an dem zu behandelnden Ort aufgeweitet und gegen die Gefäßwand gedrückt. Dieses Aufweiten kann beispielsweise mit Hilfe eines Angioplastie-Ballons, aufgebracht auf einen einführbaren Katheter, erfolgen. Alternativ sind auch selbst-expandierende Stents bekannt. Diese sind beispielsweise aus einem superelastischen Metall, wie Nitinol, aufgebaut.

Stents werden derzeit in zwei Grundtypen eingeteilt, die dauerhaften Stents und degradierbaren Stents. Dauerhafte Stents sind so ausgestaltet, dass sie im Gefäß für einen unbestimmten Zeitraum verbleiben könnten. Degradierbare Stents hingegen werden über einen vorbestimmten Zeitraum hinweg in einem Gefäß abgebaut.

Damit die Aufweitung der Gefäße durch Stent-Implantationen, sogenanntes Stenting, insbesondere auch im Rahmen eines Angioplastie-Ballon-Verfahrens erfolgreich ist, sollte ein Stent bzw. ein Angioplastie-Ballon so gewählt sein, dass es im Bereich des eingesetzten Stents nicht erneut zu Gefäßverengungen kommt.

Üblicherweise werden hierzu wirkstoffbeschichtete Stents sowie Angioplastie-Ballons verwendet, wobei die Wirkstoffe entweder den Heilungsprozess beschleunigen oder möglicherweise auftretenden Irritationen aufgrund des implantierten Stents bzw. des eingeführten Angioplastie-Ballons entgegenwirken.

Es ist deshalb wünschenswert, dass diese Wirkstoffe über einen möglichst langen Zeitraum ihre Wirkeigenschaften aufrechterhalten können, so dass die Heilung der Gefäßwand bzw. die Reduzierung der Nebenwirkungen über einen möglichst langen Zeitraum unterstützt wird.

Es ist deshalb die Aufgabe der vorliegenden Erfindung, eine verbesserte Wirkstoffbeschichtung für ein in ein Gefäß implantierbares oder einführbares Medizinprodukt bereitzustellen, wobei die Beschichtung einfach herstellbar sein soll, (im wesentlichen) keine unerwünschte physiologische Reaktionen hervorrufen soll, ausreichende mechanische Eigenschaften aufweisen soll, d. h. dass ein Stent vor dem Crimpen beschichtet werden kann und nach dem Crimpen eine (im wesentlichen) funktionstüchtige Beschichtung aufweist, und/oder die Beschichtung eine ausreichende Kratzfestigkeit aufweisen soll, damit sie insbesondere bei und nach der Einbringung in den menschlichen oder tierischen Organismus mittels eines Führungskatheters nicht beschädigt wird, und/oder die Dauer der Wirkstoffwirkung gegenüber herkömmlichen Wirkstoffbeschichtungen erhöht vorliegt.

### Zusammenfassung der Erfindung:

Die vorliegende Aufgabe wird vollständig oder teilweise durch die erfindungsgemäßen Gegenstände der unabhängigen Ansprüche gelöst.

Demnach betrifft eine erste erfindungsgemäße Ausgestaltung ein Medizinprodukt implantierbar oder einführbar in ein Gefäßsystem eines menschlichen oder tierischen Organismus mit einer Wirkstoffbeschichtung auf der Oberfläche des Medizinproduktes, d**adurch gekennzeichnet, dass**
a) die Wirkstoffbeschichtung ein oder mehrere separate Wirkstoffpartikel umfassend ein oder mehrere Wirkstoffe und ein oder mehrere Polymere mit einem Partikeldurchmesser kleiner oder gleich 30 µm umfasst oder daraus besteht,
b) das oder die Wirkstoffpartikel unter physiologischen Bedingungen im Gefäßsystem aus der Beschichtung des Medizinproduktes ablösbar sind und
c) das oder die abgelösten Wirkstoffpartikel den oder die Wirkstoffe bei einem pH-Wert im Bereich von 7,3 - 7,5 im Wesentlichen nicht freisetzen.

Eine zweite erfindungsgemäße Ausgestaltung betrifft ein Verfahren zur Herstellung eines in ein Gefäßsystem eines menschlichen oder tierischen Organismus implantierbaren oder einführbaren Medizinproduktes mit einer Wirkstoffbeschichtung, **dadurch gekennzeichnet, dass**
a) ein oder mehrere separate Wirkstoffpartikel bereitgestellt werden, die ein oder mehrere Wirkstoffe und ein oder mehrere Polymere umfassen und einen Partikeldurchmesser kleiner oder gleich 30 µm aufweisen,
b) ein Grundkörper eines Medizinproduktes bereitgestellt wird,
c) der Grundkörper aus Schritt b) mit den Wirkstoffpartikeln aus Schritt a) so beschichtet wird, dass die Wirkstoffpartikel im beschichteten Zustand unter physiologischen Bedingungen im Gefäßsystem aus der Beschichtung des Medizinproduktes ablösbar sind, aber der oder die Wirkstoffe bei einem pH-Wert im Bereich von 7,3 bis 7,5 im Wesentlichen nicht aus den abgelösten Wirkstoffpartikeln freigesetzt werden.

Eine dritte erfindungsgemäße Ausgestaltung betrifft eine Wirkstoffbeschichtung für ein in ein Gefäßsystem eines menschlichen oder tierischen Organismus implantierbares oder einführbares Medizinprodukt, **dadurch gekennzeichnet, dass**
a) die Wirkstoffbeschichtung ein oder mehrere separate Wirkstoffpartikel umfassend ein oder mehrere Wirkstoffe und ein oder mehrere Polymere mit einem Partikeldurchmesser kleiner oder gleich 30 µm umfasst oder daraus besteht,
b) das oder die Wirkstoffpartikel unter physiologischen Bedingungen im Gefäßsystem aus der Beschichtung des Medizinproduktes ablösbar sind und
c) das oder die abgelösten Wirkstoffpartikel den oder die Wirkstoffe bei einem pH-Wert im Bereich von 7,3 - 7,5 im Wesentlichen nicht freisetzen.

Eine vierte erfindungsgemäße Ausgestaltung betrifft eine Verwendung der erfindungsgemäßen Wirkstoffbeschichtung zur Herstellung eines in ein Gefäßsystem eines menschlichen oder tierischen Organismus implantierbaren oder einführbaren Medizinproduktes.

Eine fünfte erfindungsgemäße Ausgestaltung betrifft ein Verfahren zur Behandlung einer Stenose, einer calcifizierten oder weichen Plaque oder eines Aneurysma im Gefäßsystem eines menschlichen oder tierischen Organismus, **dadurch gekennzeichnet, dass** ein wirkstoffbeschichtetes erfindungsgemäßes Medizinprodukt bereitgestellt wird und in das Gefäßsystem des menschlichen oder tierischen Organismus implantiert oder eingeführt wird.

Bevorzugte Ausgestaltungen der erfindungsgemäßen Gegenstände werden in den abhängigen Ansprüchen sowie der nachfolgenden detaillierten Beschreibung dargestellt und sind, sofern sinnvoll, untereinander kombinierbar.

### Detaillierte Beschreibung der Erfindung:

Die vorliegende Erfindung beruht auf der überraschenden Erkenntnis, dass von einem erfindungsgemäßen Medizinprodukt nach Implantation oder Einführung in ein Gefäßsystem eines menschlichen oder tierischen Organismus ein oder mehrere separate Wirkstoffpartikel der erfindungsgemäßen Wirkstoffbeschichtung mit einem Partikel-Durchmesser kleiner oder gleich 30 µm vorzugsweise am Ort des Interesses, d.h. im Bereich von einer Stenose, einer calcifizierten oder weichen Plaque oder eines Aneurysma im Gefäßsystem eines menschlichen oder tierischen Organismus, aus der Beschichtung des Medizinproduktes abgelöst werden, wobei die abgelösten Wirkstoffpartikel vom Organismus als Fremdkörper erkannt und von körpereigenen Makrophagen phagozytiert werden.

Die Makrophagen bilden hierbei zunächst Pseudopodien (Plasmaausstülpungen) und umschließen damit vorzugsweise vollständig den Fremdkörper. Bei diesem Vorgang entsteht eine Vakuole, das sogenannte Phagosom. Es ist gekennzeichnet durch einen maximalen Durchmesser von ungefähr 30 µm, so dass der erfindungsgemäß zu verwendende abgelöste Wirkstoffpartikel mit einem Partikel-Durchmesser kleiner oder gleich 30 µm vorzugsweise vollständig aufgenommen werden kann. Im Phagosom herrscht üblicherweise ein pH-Wert von ungefähr 6,5. Die Vakuole fusioniert anschließend mit den Makrophagen-Lysosomen und es entsteht ein Phagolysosom, das neben reaktiven chemischen Spezies (RCS), beispielsweise NO, H₂, O₂, O₂⁻, auch lytische Enzyme enthält und in dem ein pH-Wert von ungefähr 5 herrscht.

Die erfindungsgemäß zu verwendenden Wirkstoffpartikel umfassend ein oder mehrere Wirkstoffe und ein oder mehrere Polymere sind so eingestellt, dass sie den oder die Wirkstoffe bei einem physiologischen pH-Wert im Bereich von 7,3 bis 7,5 (im Wesentlichen) nicht freisetzen. Erfindungsgemäß bedeutet "nicht freisetzen" bzw. "im Wesentlichen nicht freisetzen", dass vorzugsweise 60 Gew.% oder mehr, vorzugsweise 80 Gew.% oder mehr, weiter bevorzugt 90 Gew.% oder mehr des Wirkstoffes bezogen auf das Gewicht des Wirkstoffs im Wirkstoffpartikel nicht bei einem pH-Wert im Bereich von 7,3 - 7,5 innerhalb der ersten 24 Stunden freigesetzt werden. In einer bevorzugten Ausgestaltung werden der oder die Wirkstoffe des Wirkstoffpartikels bei einem pH-Wert, der in dem Phagosom bzw. dem Phagolysosom herrscht, d.h. bevorzugt bei pH-Wert im Bereich von 6,5 oder weniger, weiter bevorzugt bei im Bereich von 5 - 3 aus dem Wirkstoffpartikel freigesetzt.

Eine Einstellung der Freisetzungsgeschwindigkeit des oder der Wirkstoffe aus dem erfindungsgemäß zu verwendenden Wirkstoffpartikel kann weiterhin über die Löslichkeit des oder der Wirkstoffe und gegebenenfalls über die Größe des erfindungsgemäß zu verwendenden Wirkstoffpartikels vorgenommen werden. Da erfindungsgemäß der Sättigungspunkt und damit die Rate der Freisetzung des Wirkstoffes beim lysosomalen pH-Wert höher als beim cytoplasmatischen pH-Wert liegt, kann auch die Größe der erfindungsgemäß zu verwendenden Wirkstoffpartikel für die Freisetzungskinetik entscheidend sein, da für eine schnelle Freisetzung der Makrophage den Wirkstoffpartikel vorzugsweise vollständig umschließen sollte. D.h., dass je kleiner der Wirkstoffpartikel ist, üblicherweise ein umso schnellerer Abbau erfolgt. Erste in vitro Untersuchungen zeigen, dass innerhalb von wenigen Stunden bis einem Tag die Aufnahme der aus der Beschichtung abgelösten Wirkstoffpartikel in die Makrophagen erfolgt. Erfindungsgemäß setzen die Wirkstoffpartikel den oder die Wirkstoffe anschließend im Phagosom bzw. Phagolysosom frei. Deshalb liegt der durchschnittliche Partikeldurchmesser vorzugsweise im Bereich von 0,01 bis 10 µm, weiter bevorzugt 0,1 bis 5 µm und ganz besonders bevorzugt 80 bis 500 nm. Erreicht die lokale Konzentration des oder der Wirkstoffe im Phagosom bzw. Phagolysosom einen kritischen Wert, so geht das Phagosom bzw. Phagolysosom zugrunde und setzt den oder die Wirkstoffe zur Wirkung vorzugsweise am Ort des Interesses frei.

Die Makrophagen können somit neben dem Medizinprodukt selbst als weiterer Speicher für die Wirkstoffpartikel dienen. In dem Speicher werden anschließend der oder die Wirkstoffe freigesetzt, die dann direkt am Ort des Interesses wirken können. Das ist vor allem bei solchen Wirkstoffen von Interesse, die ansonsten auf Grund ihrer Hydrophilie eine zu kurze Verweildauer im Gewebe aufweisen. Deshalb kann es erfindungsgemäß gegenüber herkömmlich wirkstoffbeschichteten Medizinprodukten zu einer Verlängerung und/oder Erhöhung der lokalen Konzentration der Wirkstoffe insbesondere am Ort des Interesses kommen, da nach Beendigung der Freisetzung der Wirkstoffpartikel aus der Beschichtung des Medizinproduktes zunächst die Wirkstoffpartikel in den Makrophagen gespeichert werden und erst dort den oder die Wirkstoffe freisetzen. Bei herkömmlich wirkstoffbeschichteten Medizinprodukten werden im Gegensatz zur vorliegenden Erfindung üblicherweise die Wirkstoffe direkt aus der Wirkstoffbeschichtung freigesetzt und regelmäßig direkt mit dem Blutstrom abtransportiert, so dass die Wirkdauer und die Wirkkonzentration gegenüber der vorliegenden Erfindung herabgesetzt sein kann.

Ein erfindungsgemäß einzusetzender Wirkstoffpartikel ist vorzugsweise so aufgebaut, dass das oder die Polymere ausgewählt werden aus pH-sensitiv degradierbaren Polymeren. In einer weiteren bevorzugten Ausgestaltung sind der oder die Wirkstoffe von einem pH-sensitiv degradierbaren Polymer(gemisch) umhüllt (verkapselt) oder liegen in dessen Matrix inkorporiert vor. In einer besonders bevorzugten Ausgestaltung liegen sie verkapselt vor. Derart bevorzugte Wirkstoffpartikelausgestaltungen ermöglichen durch die Auswahl eines oder mehrerer entsprechend geeigneten pH-sensitiv degradierbaren Polymere, dass der oder die Wirkstoffe bei einem pH-Wert im Bereich von 7,3 - 7,5 im Wesentlichen nicht (bzw. langsam), aber bei einem pH-Wert im Bereich von 6,5 oder weniger, vorzugsweise 5 bis 3 (beschleunigt) freigesetzt werden. Geeignete Polymermaterialien degradieren entsprechend bei einem pH-Wert im Bereich von 6,5 oder weniger, bevorzugt 5 bis 3 gegenüber einem pH-Wert im Bereich von 7,3 - 7,5 beschleunigt. Sie werden dementsprechend besonders bevorzugt ausgewählt aus der Gruppe bestehend aus Poly(β-aminoestern), Poly(ethylenoxid) modifizierten Poly(β-aminoestern), Polylactiden, Poly(lactid-co-glycoliden), modifizierten Cyclodextrinen, modizifizierten Kohlenhydraten und/oder sonstigen Polyestern.

Erfindungsgemäß geeignete Poly(β-aminoester)-Wirkstoffpartikel sowie deren Herstellverfahren werden beispielsweise durch David M. Lynn et al. ("pH-Responsive Polymer Microspheres: Rapid Release of Encapsulated Material within the Range of Intracellular pH", Angew. Chem. Int. Ed.; 2001; 40; No. 9; S. 1707-1710) beschrieben.

Erfindungsgemäß geeignete Poly(ethylenoxid)-modifizierte-Poly(β-aminoester)-Wirkstoffpartikel sowie deren Herstellverfahren werden u.a. in Shenoy et al. ("Poly(Ethylen Oxide)-modified Poly(β-Amino-Ester) Nanoparticles as a pH-Sensitive System for Tumor-Targeted Delivery of Hydrophobic Drugs: Part 1. In vitro Evaluations", Mol Pharm. 2005; 2(5): 357-366, siehe auch ("Poly(Ethylen Oxide)-modified Poly(β-Amino-Ester) Nanoparticles as a pH-Sensitive System for Tumor-Targeted Delivery of Hydrophobic Drugs: Part 2. In vivo Distribution and Tumor Localization Studies", Pharm Res.; 2005 December; 22(12): 2107-2114) sowie durch A. Potineni et al. ("Poly(ethylene oxide)-modified poly(β-amino-ester) nanoparticles as a pH-sensitive biodegradable system for paclitaxel delivery", J. Controlled Release; 86 (2003); 223-234) beschrieben.

Erfindungsgemäß geeignete Polylactid- bzw. Poly(lactid-co-glycolid)-Wirkstoffpartikel sowie deren Herstellverfahren werden beispielsweise durch H. Fessi et al. ("Nanocapsule formation by interfacial polymer deposition following solvent displacement", Int. J. Pharmaceutics; 55 (1989); R1-R4), M. Konerackä et al. ("Encapsulation of anticancer drug and magnetic particles in biodegradable polymer nanospheres", J. Phys.: Condens. Matter; 20 (2008); 204151 (6pp)), D. Klose et al. ("PLGA-based drug delivery systems: Importance of the type of drug and device geometry"; Int. J. Pharmaceutics; 354 (2008); 95-103) und Kang et al. ("Preparation of PLLA/PLGA microparticles using solution enhanced dispersion by supercritical fluids (SEDS)", J. Colloid and Interface Science; 322 (2008); 87-94) beschrieben.

Erfindungsgemäß geeignete Cyclodextrinwirkstoffpartikel sowie deren Herstellverfahren werden beispielsweise durch Erem Memisoglu et al. ("Amphiphilic β-Cyclodextrins Modified on the Primary Face: Synthesis, Characterization, and Evaluation of Their Potential as Novel Excipients in the Preparation of Nanocapsules"; J. Pharm. Sciences; Vol. 91; No. 5; May 2002) sowie Erem Bilensoy et al. ("Safety and efficacy of amphiphilic βcyclodextrin nanoparticles for Paclitaxel delivery"; Int. J. Pharmaceutics; 347 (2008); 163-170) beschrieben.

Die oben beispielhaft aufgeführten erfindungsgemäß einzusetzenden Wirkstoffpartikel sind besonders geeignet für die Verwendung von Wirkstoffen mit hydrophoben Oberflächeneigenschaften.

Die erfindungsgemäß einzusetzenden Wirkstoffpartikel werden mittels üblicher Verfahren auf die Oberfläche des Medizinproduktes aufgetragen. Hierbei können die Wirkstoffpartikel bevorzugt (a) inkorporiert in eine degradierbare Matrix oder (b) mittels einer Haftschicht auf der Oberfläche des Medizinproduktes als Wirkstoffbeschichtung vorliegen, um unter physiologischen Bedingungen, vorzugsweise bei einem pH-Wert im Bereich von 7,0 bis 7,5 aus der Wirkstoffbeschichtung des Medizinproduktes ablösbar zu sein.

Liegen der oder die Wirkstoffpartikel (a) inkorporiert in eine degradierbaren Matrix vor, so werden sie aufgrund von Erosion/Degradierung des Matrixmaterials Schritt für Schritt freigesetzt. Folglich werden die Materialien der degradierbaren Matrix so ausgewählt, dass sie im Vergleich zu den Polymermaterialien des Wirkstoffpartikels unter physiologischen Bedingungen, vorzugsweise bei einem pH-Wert im Bereich von 7,0 bis 7,5 schneller degradieren bzw. erodieren. Gemäß dieser erfindungsgemäß bevorzugten Ausgestaltung bildet die degradierbare Matrix ein erstes Speichermedium für die Wirkstoffpartikel und die Makrophagen ein zweites Speichermedium. Mittels dieser bevorzugten Ausgestaltung kann die Wirkdauer des oder der Wirkstoffe weiterhin verlängert werden. In einer weiteren bevorzugten Ausgestaltung hiervon wird die degradierbare Matrix ausgewählt aus ein oder mehreren Materialien aus der Gruppe bestehend aus Polydioxanon; Polycaprolacton, Polyglycoliden; Polylactiden, vorzugsweise Poly(L-lactid), Poly(D-lactid), Poly(D,L-lactid) sowie Blends, Co-Polymere und Tri-Polymere hiervon, vorzugsweise Poly(L-lactid-co-glycolid), Poly(D-L-lactid-co-glycolid), Poly(L-lactid-co-L-lactid), Poly(L-lactid-co-trimethylencarbonat); Polysacchariden, vorzugsweise Chitosan, Levan, Hyaluronsäure, Heparin, Dextran und Cellulosen; Polyhydroxyvalerat; Ethylvinylacetat; Polyethylenoxiden; Polyphosphorylcholin; Fibrin; Albumin; und/oder Polyhydroxybuttersäuren, vorzugsweise ataktische, isotaktische und/oder syndiotaktische Polyhydroxybuttersäure sowie deren Blends.

Werden der oder die Wirkstoffpartikel (b) mittels ein oder mehrerer Haftmaterialien auf die Oberfläche des Medizinproduktes unter physiologischen Bedingungen ablösbar beschichtet, so wird vorzugsweise die Bindung zwischen Wirkstoffpartikel und Haftmaterial unter physiologischen Bedingungen, vorzugsweise bei einem pH-Wert im Bereich von 7,0 bis 7,5 schneller aufgelöst (üblicherweise degradiert das Haftmaterial) als das Polymer der Wirkstoffpartikel degradiert und den oder die Wirkstoffe freisetzt. Üblicherweise ist die Bindung des Haftmaterials an den Wirkstoffpartikeln jedoch so stark, dass bei Implantation oder Einführen des Medizinproduktes die erfindungsgemäße Wirkstoffbeschichtung auf dem erfindungsgemäßen Medizinprodukt nicht bzw. im Wesentlichen nicht funktionell beeinträchtigt wird. Entsprechend sind als Haftmaterialien alle physiologisch geeigneten Materialien bevorzugt, welche unter physiologischen Bedingungen schneller degradieren als das Polymer im Wirkstoffpartikel. Besonders bevorzugte Haftmaterialien werden ausgewählt aus der Gruppe bestehend aus Kohlenhydraten, vorzugsweise Zucker und/oder Polysacchariden, vorzugsweise Stärken und/oder Hyaluronsäure; (Soja-)Lecithin; (Oligo-)Peptiden und/oder schnell degradierbaren Polyestern.

Ein Wirkstoff im Sinne dieser Erfindung ist eine Substanz oder eine Verbindung, die im menschlichen oder tierischen Körper eine biologische Reaktion hervorruft. In diesem Sinne kann der Begriff Wirkstoff auch synonym zu Arzneistoff und/oder Pharmakon verwendet werden. Im Sinne der vorliegenden Erfindung ist das erfindungsgemäße Medizinprodukt, vorzugsweise Stent oder Angioplastie-Ballon mit ein oder mehreren Wirkstoffen in einer Konzentration beschichtet, die ausreicht, die gewünschten physiologischen Reaktionen hervorzurufen.

Erfindungsgemäß zu verwendende Wirkstoffe werden bevorzugt ausgewählt aus der Gruppe bestehend aus Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statinen, vorzugsweise Simvastatin, Mevastatin, Atorvastatin, Lovastatin, Pitavastatin und Fluvastatin; Estrogenen, vorzugsweise 17b-Estradiol, Daizein und Genistein; Lipidregulatoren, vorzugsweise Fibrate; Immunsuppressiva; Vasodilatatoren, vorzugsweise Satane; Calciumkanalblocker; Calcineurininhibitoren, vorzugsweise Tacrolimus; Antiinflammatorika, vorzugsweise Imidazole; Antiallergika; Oligonucleotiden, vorzugsweise Decoy-Oligodesoxynukleotid (dODN); Endothelbildner, vorzugsweise Fibrin; Steroiden; Proteinen/Peptiden; Proliferationshemmer; Analgetika und Antirheumatika; Endothelinrezeptor-Antagonisten, vorzugsweise Bosentan; Rho-Kinase Inhibitoren, vorzugsweise Fasudil; RGD-Peptiden (umfassend die Sequenz Arg-Gly-Asp); und/oder organischen Goldverbindungen.

Im Sinne der vorliegenden Erfindung können Medizinprodukte in ein Gefäßsystem eines menschlichen oder tierischen Körpers (i) implantierbar oder (ii) einführbar sein.

Übliche (i) implantierbare Medizinprodukte (Implantate bzw. Implantatgrundkörper) können alle medizinischen, plastischen und/oder funktionellen Implantate bzw. Implantatgrundkörper darstellen, die in ein Gefäß eines menschlichen oder tierischen Organismus implantiert werden und Wirkstoffe freisetzen (eluieren). Besonders bevorzugt ist ein Wirkstoff-eluierender Stent als ein erfindungsgemäßes Medizinprodukt.

Üblicherweise sollen die ursprünglich mechanischen Funktionen der erfindungsgemäßen implantierbaren Medizinprodukte beibehalten werden, d.h. bei einem Koronarstent bspw. die Dilatierbarkeit, der geringe Recoil, die Stabilität über eine gewünschte Zeitdauer (im Falle eines degradierbaren Stents) sowie die Flexibilität.

Im Folgenden werden erfindungsgemäß üblicherweise zu verwendende Implantat-Materialien, bevorzugt Stentgrundkörper-Materialien sowie bevorzugte Ausgestaltungen hiervon beschrieben:

### Degradierbarer Implantat-Grundkörper, insbesondere degradierbarer Stentgrundkörper:

Im Sinne der vorliegenden Erfindung bedeutet "degradierbarer Implantat(grundkörper)", insbesondere "degradierbarer Stent(grundkörper)", dass der Grundkörper in physiologischer Umgebung, insbesondere im Gefäßsystem eines menschlichen oder tierischen Organismus degradiert, d. h. so abgebaut wird, dass der Stent seine Integrität verliert. Vorzugsweise werden degradierbare Implantatgrundkörper erst dann abgebaut, wenn die Funktion des Implantats nicht mehr physiologisch sinnvoll bzw. notwendig ist. Bei degradierbaren Stents bedeutet das, dass der Stent vorzugsweise erst dann degradiert oder seine Integrität verliert, wenn das traumatisierte Gewebe des Gefäßes verheilt ist und der Stent somit nicht länger im Gefäßlumen verbleiben muss.

### Metallischer Grundkörper:

In einer erfindungsgemäßen Ausgestaltung umfasst oder besteht der degradierbare Werkstoff bevorzugt aus einem metallischen Werkstoff, der eine bio-korrodierbare Legierung darstellt, wobei die Hauptkomponente der Legierung ausgewählt wird aus der Gruppe Magnesium, Eisen, Zink und/oder Wolfram; insbesondere wird für einen degradierbaren metallischen Werkstoff eine Magnesium-Legierung oder eine Eisen-Legierung bevorzugt, besonders bevorzugt eine Magnesium-Legierung.

Die Legierung, insbesondere umfassend Magnesium, Eisen, Zink und/oder Wolfram ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau stattfindet, der letztendlich dazu führt, dass der aus dem Werkstoff gebildeten Teil des Stents seine mechanische Integrität verliert. Unter Legierung wird vorliegend ein metallisches Gefüge verstanden, dessen Hauptkomponente Magnesium, Eisen, Zink und/oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, weiter bevorzugt mehr als 70 Gew.%. Eine Magnesium-Legierung oder eine Eisen-Legierung ist bevorzugt. Besonders bevorzugt ist eine Magnesium-Legierung.

Ist der Werkstoff eine Magnesium-Legierung, so enthält diese vorzugsweise Yttrium und weitere Seltenerdmetalle, da sich eine derartige Legierung aufgrund ihrer physikochemischen Eigenschaften und ihrer hohen Bio-Kompatibilität, insbesondere auch ihrer Abbauprodukte, auszeichnet.

Besonders bevorzugt werden Magnesium-Legierungen der WE-Reihe, insbesondere WE43, sowie Magnesium-Legierungen der Zusammensetzung Seltenerdmetalle 5,5 - 9,9 Gew.%, davon Yttrium 0,0 - 5,5 Gew.% und Rest < 1 Gew.%, wobei der Rest Zirkonium und/oder Silicium enthalten kann und wobei Magnesium den auf 100 Gew.% fehlenden Anteil an der Legierung einnimmt, eingesetzt. Diese Magnesium-Legierungen bestätigten bereits experimentell und in ersten klinischen Versuchen ihre besondere Eignung, d. h. sie zeigten eine hohe Bio-Kompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 an Lanthan (57) folgenden Elemente, nämlich Cer (58), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

### Polymer-Grundkörper:

Implantat(grundkörper), insbesondere Stent(grundkörper) können gemäß einer weiteren alternativen erfindungsgemäßen Ausgestaltung degradierbares Polymer umfassen oder daraus bestehen, vorzugsweise ausgewählt aus der Gruppe bestehend aus: Polydioxanon; Polycaprolacton, Polyhydroxyvaleriansäure; Polyhydroxybuttersäure; Polyglycoliden; Polylactiden, vorzugsweise Poly(L-lactid), Poly(D-lactid), Poly(D,L-lactid) sowie Blends, Co-Polymere und Tri-Polymere hiervon, vorzugsweise Poly(L-lactid-co-glycolid), Poly(D-L-lactid-co-glycolid), Poly(L-lactid-co-L-lactid), Poly(L-lactid-co-trimethylencarbonat); Polysacchariden, vorzugsweise Chitosan, Levan, Hyaluronsäure, Heparin, Dextran und Cellulosen; Phosphazene; Polyphosphoester; Polyphosphonate und Polyphosphite.

### Dauerhafter Implantat(grundkörper), vorzugsweise dauerhafter Stent(qrundkörper):

Im Gegensatz zu degradierbaren Grundkörpern wird der dauerhafte Implantat(grundkörper), insbesondere der dauerhafte Stent(grundkörper) in physiologischer Umgebung im menschlichen oder tierischen Organismus im Wesentlichen nicht abgebaut, d. h., dass er seine Integrität behält.

In einer weiteren erfindungsgemäßen Ausgestaltung umfasst oder besteht der Grundkörper eines dauerhaften Implantats, insbesondere eines dauerhaften Stents vorzugsweise aus einem Formgedächtnis-Material mit ein oder mehreren Materialien ausgewählt aus der Gruppe der Nickel-Titan-Legierungen und Kupfer-Zink-Aluminium-Legierungen, vorzugsweise aus Nitinol.

In einer besonders bevorzugten erfindungsgemäßen Ausgestaltung umfasst oder besteht der Grundkörper eines dauerhaften Implantats, insbesondere eines dauerhaften Stents aus Edelstahl, vorzugsweise aus einem Cr-Ni-Fe-Stahl, hier bevorzugt die Legierung 316L, oder einem Co-Cr-Stahl.

In einer weiteren bevorzugten Ausgestaltung kann der Grundkörper des Implantates, vorzugsweise Stents, zusätzlich Kunststoff, vorzugsweise Polyurethan, und/oder Keramik und/oder weitere Polymerbeschichtungen umfassen.

Werden im Sinne der vorliegenden Erfindung endovaskulär implantierbare Stents als implantierbare Medizinprodukte verwendet, so können alle üblichen Stentgeometrien verwendet werden. Insbesondere bevorzugt sind Stentgeometrien, die insbesondere in US 6,896,695, US 2006/241742, US 5,968,083 (Tenax), EP 1 430 854 (Helix-Design), US 6, 197,047 und EP 0 884 985 beschrieben werden.

Ein erfindungsgemäßer peripherer oder koronarer Stent wird vorzugsweise auf der muralen Seite mit der erfindungsgemäßen Wirkstoffbeschichtung beschichtet, d.h. bei einer üblicherweise zylinderförmigen Stentgeometrie, die Oberfläche, die mit dem Gewebe und nicht dem Gefäßlumen des Blutgefäßes nach Implantation in Kontakt steht. Eine solche erfindungsgemäß bevorzugte Beschichtung kann dazu beitragen, dass weniger Nebenwirkungen hervorgerufen werden. Bei einem degradierbaren Grundkörper erlaubt diese bevorzugte Beschichtung zudem den Abbau von der luminalen Oberfläche des Stents, d.h. der Oberfläche, die bei einem üblicherweise zylinderförmigen Stent mit dem Lumen des Gefäßes in Kontakt steht.

In einer weiteren erfindungsgemäßen Ausgestaltung stellen (ii) einführbare Medizinprodukte vorzugsweise Wirkstoff-eluierende Angioplastie-Ballons dar. Hierzu werden übliche, serienmäßige Ballonkatheter verwendet, die bevorzugt "compliant sind, d.h. deren Durchmesser sich also mit zunehmendem Druck über einen relativ großen Bereich verändert. Diese üblichen Ballonkatheter werden erfindungsgemäß beschichtet, um ein erfindungsgemäßes einführbares Medizinprodukt zu erhalten.

In einer weiteren bevorzugten Ausgestaltung kann ein erfindungsgemäßes Medizinprodukt auf der erfindungsgemäß zu verwendenden Wirkstoffbeschichtung zusätzlich eine oder mehrere weitere (wirkstofffreie) Beschichtungen als sogenannten "Top-Coat" aufweisen, um insbesondere das Risiko einer Abrasion (eines Teils) der Wirkstoffbeschichtung bei der Implantation oder Einführung des Medizinproduktes in das Gefäßsystem zu verringern.

Für den Top-Coat können erfindungsgemäß üblicherweise ein oder mehrere Polymere verwendet werden, die bevorzugt ausgewählt werden aus der Gruppe bestehend aus:
- nicht degradierbare Polymere: Polyethylene; Polyvinylchloride, Polyvinylfluoride, Polyvinylalkohole; Polyacrylate, vorzugsweise Polyethyl- und Polymethacrylate; Polymethylmetacrylate, Polymethyl-co-Ethylacrylate und Ethylen/Ethylacrylate; Polytetrafluorethylen, vorzugsweise Ethylen/Chlortrifluorethylen-co-Polymere, Ethylen/Tetrafluorethylen-Co-Polymere; Polyamide, vorzugsweise Polyamidimid, PA-11,PA-12, PA-46, PA-66; Polyetherimide; Polyethersulfone; Poly(iso)butylene; Polyurethane; Polybutylenterephthalate; Silikone, Polyphosphatane; Polymerschäume, vorzugsweise Polymerschäume aus Carbonaten, Styrolen; Co-Polymere und/oder Blends der Polymerklassen, Polymere der Klasse der Thermoplaste und/oder
- degradierbare Polymere: Polydioxanone; Polycaprolactone, Polyhydroxyvaleriansäure(-Derivate); Polyglycolide; Polylactide, vorzugsweise Poly(L-lactid), Poly(D-lactid), Poly(D,L-lactid) sowie Blends, Co-Polymere und Tri-Polymere hiervon, vorzugsweise Poly(L-lactid-co-glycolid), Poly(D-L-lactid-co-glycolid), Poly(L-lactid-co-L-lactid), Poly(L-lactid-co-trimethylencarbonat); Ethylvinylacetat; Polyethylenoxide, Polyphosphorylcholine; Fibrin; Albumin; Polyhydroxybuttersäure, vorzugsweise ataktische, isotaktische und/oder syndiotaktische Polyhydroxybuttersäure sowie deren Blends.

Besonders bevorzugte Polymere für den "Top-Coat" der vorliegenden Erfindung sind die oben beschriebenen degradierbaren Polymere, da durch den vollständigen Abbau des oder der Polymere kein körperfremder Bestandteil im Organismus verbleibt.

Gemäß der zweiten erfindungsgemäßen Ausgestaltung wird ein Verfahren zur Herstellung eines in ein Gefäß eines menschlichen oder tierischen Organismus implantierbaren oder einführbaren Medizinproduktes mit einer Wirkstoffbeschichtung beansprucht. Vorzugsweise wird ein Verfahren zur Herstellung eines erfindungsgemäßen Medizinproduktes beansprucht. Die bevorzugten Ausgestaltungen zu dem erfindungsgemäßen Medizinprodukt bzw. der erfindungsgemäßen Wirkstoffbeschichtung finden auch auf das Verfahren zur Herstellung des erfindungsgemäßen Medizinproduktes Anwendung und können in jeder für den Fachmann sinnvollen Art und Weise kombiniert werden.

Bezüglich der erfindungsgemäßen Ausgestaltung zum Herstellverfahren werden gemäß Verfahrensschritt a) erfindungsgemäß einzusetzende Wirkstoffpartikel bereitgestellt. Entsprechende Verfahren zur Herstellung von erfindungsgemäß zu verwendenden Wirkstoffpartikel sind dem Fachmann bekannt und sind zudem beispielhaft bereits oben beschrieben worden. Hierbei beschreiben Kang et al. (ibid.) und Potineni et al. (ibid.) die Herstellung von Nanopartikeln umfassend einen Wirkstoff umhüllt in einem Polymer aus Superkritischen Flüssigkeiten. Die Herstellung von Wirkstoff-Nanopartikeln durch Ausfällen in Wasser wird beispielsweise in Bilensoy et al. beschrieben.

Gemäß Schritt b) des erfindungsgemäßen Herstellverfahrens wird ein Grundkörper des Medizinproduktes bereitgestellt. Erfindungsgemäß einzusetzende Grundkörper wurden bereits zu den erfindungsgemäßen Medizinprodukten, insbesondere Implantate und Implantat-Grundkörper, vorzugsweise Stents bzw. Stentgrundkörper, sowie den einführbaren Medizinprodukten, bevorzugt Angioplastie-Ballons beschrieben. Als Implantat-Grundkörper wird besonders bevorzugt ein Stentgrundkörper und hierbei insbesondere ein degradierbarer Stentgrundkörper, wie bereits oben beschrieben, verwendet.

Gemäß Schritt c) des erfindungsgemäßen Herstellverfahrens wird der Grundkörper aus Schritt b) mit den Wirkstoffpartikeln aus Schritt a) so beschichtet, dass der oder die Wirkstoffpartikel im beschichteten Zustand unter physiologischen Bedingungen, vorzugsweise bei einem pH-Wert im Bereich von 7,0 - 7,5 im Gefäßsystem aus der Beschichtung ablösbar sind.

Eine solche erfindungsgemäße Wirkstoffbeschichtung kann gemäß üblicher Verfahren bewerkstelligt werden, wobei hierzu vorteilhaft ein Wirkstoffpartikel-Trockengemisch, ein Wirkstoffpartikel-Lösungsmittel-Bindemittel-Gemisch (Lösungsmittel ist geeignet das Bindemittel (an)zulösen, aber nicht die Materialien der Wirkstoffpartikel) oder ein Wirkstoffpartikel-Polymergemisch, beispielsweise mittels eines Tauchverfahrens (Dip-Coating), einer Sprühbeschichtung mittels Ein- bzw. Mehrstoffdüse, Rotationszerstäubung und Sputtern auf die gegebenenfalls vorbehandelte Oberfläche des Medizinproduktes aufgetragen wird.

Wird erfindungsgemäß ein Wirkstoffpartikel-Trockengemisch verwendet, so wird vor der Beschichtung des Medizinproduktes mit den erfindungsgemäß einzusetzenden Wirkstoffpartikeln die entsprechende Oberfläche des Medizinproduktes mit einem Haftmaterial so vorbehandelt, dass in einem nachfolgenden Schritt die erfindungsgemäß einzusetzenden Wirkstoffpartikel so an die Oberfläche des Medizinproduktes anhaften, dass die Wirkstoffpartikel unter physiologischen Bedingungen, vorzugsweise bei einem pH-Wert im Bereich von 7,0 - 7,5 von der Oberfläche des Medizinproduktes abgelöst werden. Bei einem Sprühverfahren weisen die erfindungsgemäß einzusetzenden Wirkstoffpartikel üblicherweise einen Partikeldurchmesser auf, der kleiner ist als der Düsendurchmesser. Gleiche Beschichtungsverfahren können auch üblicherweise für die Beschichtung mit dem "Top-Coat" angewendet werden.

Für den Fall, dass vorzugsweise nur die murale Oberfläche eines erfindungsgemäßen Stents mit einer erfindungsgemäßen Wirkstoffbeschichtung beschichtet werden soll, kann dies vorzugsweise dadurch bewerkstelligt werden, dass in den vorgenannten Verfahrensschritten der Stent auf beispielsweise einen Zylinder, eine Kanüle oder einen Dorn aufgesteckt wird, damit nur die murale Oberfläche des Stents mit der erfindungsgemäßen Wirkstoffbeschichtung beschichtet wird. Alternativ könnte die murale Oberfläche mit der erfindungsgemäßen Wirkstoffbeschichtung mittels eines Walzenauftrages oder durch selektives Auftragen durch Bestreichen, Befüllen von Kavitäten etc. vorgenommen werden.

Gleiche Verfahren können auch bevorzugt für die "Top-Coat-Beschichtungen" verwendet werden.

Gegebenenfalls kann einem oder mehreren Beschichtungsschritten ein üblicher Trocknungsschritt oder andere übliche physikalische oder chemische Nachbearbeitungsschritte, z. B. Vakuum- oder Plasmabehandlungen, folgen, bevor das erfindungsgemäße Medizinprodukt, vorzugsweise Stent oder Angioplastie-Ballon, weiter behandelt wird.

Die vierte erfindungsgemäße Ausgestaltung, nämlich die Verwendung der erfindungsgemäßen Wirkstoffbeschichtung zur Herstellung eines in ein Gefäßsystem eines menschlichen oder tierischen Organismus implantierbaren oder einführbaren Medizinproduktes, ist vorzugsweise auf die Verwendung der erfindungsgemäßen Wirkstoffbeschichtung zur Herstellung eines erfindungsgemäßen Medizinproduktes gerichtet. Die bevorzugten Ausgestaltungen zu dem erfindungsgemäßen Medizinprodukt bzw. der erfindungsgemäßen Wirkstoffbeschichtung gelten dementsprechend auch für die vierte Ausgestaltung und sind - sofern für einen Fachmann sinnvoll - miteinander kombinierbar.

In einer bevorzugten Ausgestaltung wird die erfindungsgemäße Wirkstoffbeschichtung oder das erfindungsgemäße Medizinprodukt verwendet, um die Wirkdauer eines oder mehrerer Wirkstoffe im Gefäßsystem eines menschlichen oder tierischen Organismus zu verlängern. Auch hier sind die bevorzugten Ausgestaltungen des erfindungsgemäßen Medizinproduktes bzw. der erfindungsgemäßen Wirkstoffbeschichtung auf deren Verwendung anwendbar und soweit sinnvoll kombinierbar.

Gemäß der fünften erfindungsgemäßen Ausgestaltung wird ein Behandlungsverfahren beansprucht, dass dadurch gekennzeichnet ist, dass ein erfindungsgemäß wirkstoffbeschichtetes Medizinprodukt bereitgestellt wird und in das Gefäßsystem des menschlichen oder tierischen Organismus implantiert oder eingeführt wird. Auch hier sind die bevorzugten Ausgestaltungen des erfindungsgemäßen Medizinproduktes bzw. der erfindungsgemäßen Beschichtung anwendbar und - sofern für einen Fachmann sinnvoll - kombinierbar.

Die bevorzugten Ausgestaltungen des erfindungsgemäß verwendbaren Medizinproduktes, vorzugsweise des erfindungsgemäßen Stents oder Angioplastie-Ballons sowie der erfindungsgemäßen Wirkstoffbeschichtung können in allen für einen Fachmann sinnvollen Varianten untereinander, aber auch mit den weiteren hierin offenbarten bevorzugten Ausgestaltungen kombiniert werden.

### Ausführungsbeispiele:

Die vorliegende Erfindung wird im Folgenden durch Ausführungsbeispiele beschrieben, die jedoch den Schutzumfang der erfindungsgemäßen Gegenstände nicht limitieren.

### A) Herstellung erfindungsgemäß zu verwendender Wirkstoffpartikel:

### Herstellungsbeispiel A1: Herstellung von Paclitaxel-beladenen Cyclodextrin-Nanopartikeln

Es wird eine Lösung aus 3 g 6-0-CAPRO-β-CD [Heptakis(6-deoxy-6-hexanamido)cyclomaltoheptanose; MW: 1820g/mol] und 640 mg Paclitaxel (MW: 854g/mol) in 1000 ml Aceton in einem 1:1 molaren Verhältnis bei Raumtemperatur (RT) unter ständigem Rühren hergestellt. Diese organische Lösung wird langsam unter Rühren in 2000 ml ultrareines Wasser gegeben, wobei die klare Lösung sofort ein milchiges Aussehen bekommt. Im Vakuum wird das Aceton entfernt, wobei die Nanopartikel in einer Größe von ca. 150-500 nm vollständig ausfallen. Diese werden abzentrifugiert, mit Pufferlösung gespült, im Vakuum getrocknet und bei 8°C im Kühlschrank gelagert.

### Herstellungsbeispiel A2: Herstellung von Sirolimus-beladenen PLGA-Nanopartikeln

Es werden 10 g PLGA [Poly(D,L-lactid-co-glycolid), Glycolidanteil 85:15; MW: 50.000-75.000; Fa. Sigma] und 200 mg Sirolimus (MW: 914g/mol; Fa. Sigma) in 1000 ml Aceton bei RT unter ständigem Rühren gelöst, um die organische Phase herzustellen. Die wässerige Phase besteht aus 10 g Pluronic^{®}F68 (Fa. BASF) als Tensid und 2000 ml hochreinem Wasser. Die organische Phase wird unter Rühren tropfenweise zur wässrigen Lösung gegeben, wobei eine kolloidale Lösung entsteht. Das organische Lösungsmittel und die Hälfte des Wasser werden unter reduziertem Druck entfernt, die ausgefallenen Nanopartikel abzentrifugiert, mit einer wässrigen Pufferlösung gespült und gefriergetrocknet.

### Herstellungsbeispiel A3: Herstellung von Bosentan-beladenen PEO-modifizierten PbAE-Nanopartikeln

Eine PEO modifizierter Poly(β-aminoester) (PbAE)-Lösung wird entsprechend der Vorschrift von Lynn et al. (ibid.) hergestellt. Die organische Phase wird durch Lösen einer äquimolaren Menge Bosentan (ein Endothelinrezeptotantagonist; Tracleer^{®}) und PEO-PbAE in absolutem Alkohol derart hergestellt, dass eine 1 mM-Lösung entsteht. Die organische Phase wird tropfenweise bei 15°C in das doppelte Volumen einer wässrigen Lösung aus 0,1-1 Gew.% Pluronic^{®}F108 (Fa. BASF) in hochreinem Wasser gegeben, wobei eine kolloidale Lösung entsteht. Das organische Lösungsmittel und die Hälfte des Wassers werden unter reduziertem Druck entfernt, die ausgefallenen Nanopartikel abzentrifugiert (10.000 U/min; 20 Min.), mit einer wässrigen Pufferlösung gespült und gefriergetrocknet.

### Herstellungsbeispiel A4: Herstellung von Paclitaxel beladenen PLA-Nanopartikeln durch Dispersion in einer superkritischen Flüssigkeit

Es werden 5 g PLGA [Poly(D,L-lactid-co-glycolid), Glycolidanteil 85:15; MW: 50.000-75.000; Fa. Sigma] und 200 mg Sirolimus (MW: 914g/mol; Fa. Sigma) in 1000 ml Dichlormethan bei RT unter ständigem Rühren gelöst. Die Polymerlösung wird gemäß der Vorschrift von Kang et al. (ibid.) durch eine HPLC Pumpe in ein Hochdruckgefäß mit überkritischem CO₂ gepumpt, wobei die Nanopartikel sofort ausfallen.

### B) Erfindungsgemäße Beschichtung eines Medizinproduktes mittels eines Sprühverfahrens

Bei der Beschichtung eines erfindungsgemäß einzusetzenden Medizinproduktes, vorzugsweise eines handelsüblichen Stents oder Angioplastie-Ballons, können die erfindungsgemäß zu verwendenden Wirkstoffpartikel, bevorzugt die unter A) hergestellten Nanopartikel, zusammen mit einem Bindemittel in einem geeigneten Lösungsmittel auf die Oberfläche des Medizinproduktes aufgebracht werden, wobei das Lösungsmittel zwar das Bindemittel (an)lösen kann, aber nicht die Wirkstoffpartikel, bevorzugt Nanopartikel. Geeignet als Lösungsmittel sind üblicherweise alle wasserlöslichen Polymere und Oligomere, wie beispielsweise Kohlenhydrate, vorzugsweise Zucker und Polysaccharide, vorzugsweise, Hyaluronsäure; und/oder Gelatine.

Des Weiteren ist zu beachten, dass die erfindungsgemäß zu verwendenden Wirkstoffpartikel, bevorzugt Nanopartikel, nicht die Düse der Beschichtungsapparatur verstopfen, d.h., dass der maximale Teilchendurchmesser der erfindungsgemäß zu verwendenden Wirkstoffpartikel bevorzugt kleiner ist als die Spaltbreite der Düse der Beschichtungsapparatur.

### Herstellungsbeispiel B1:

Es wird eine Suspension der gemäß Herstellverfahren A) hergestellten Wirkstoffbeladenen Nanopartikel (< 200 nm) in hochreinem Wasser (10 Gew.%) bei RT angesetzt. Gegebenfalls kann ein Netz- oder Dispergieradditiv (0,5-3 Gew%) wie Lecithin, AOT (Bis(2-ethyl-I-hexyl)sulfosuccinat), Polyethylenoxid-Polypropylenoxid-Block-Copolymere, Tetraalkylammoniumsalze oder Silikate zugesetzt werden.

Ein bereitgestellter Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems (Fa. EFD oder Spraying System) wird der sich drehende Stent unter konstanten Umgebungsbedingungen (Raumtemperatur; 42% Luftfeuchte) halbseitig mit der Nanopartikelsuspension beschichtet. Bei einem Düsenabstand von 20 mm ist ein 18 mm langer Stent nach ca. 10 min üblicherweise beschichtet. Nach Erreichen der beabsichtigten Schichtmasse wird der Stent 5 min im Vakuum getrocknet, ehe nach Drehen des Stents und erneutem Einspannen die unbeschichtete Seite auf dieselbe Weise beschichtet wird. Der fertig beschichtete Stent wird für 36 h bei 40°C in einem Vakuumofen (Vakucell; Fa. MMM) getrocknet.

### Herstellungsbeispiel B2:

Ein bereitgestellter Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems (Fa. EFD oder Spraying System) wird der sich drehende Stent unter konstanten Umgebungsbedingungen (Raumtemperatur; 42% Luftfeuchte) halbseitig mit einem organischen Haftmaterial besprüht. Die Wirkstoff-Nanopartikel werden anschließend mittels eines lösungsmittelfreien Verfahrens in einem Airbrush-System als trockenes Pulver auf die murale Seite des Stents gesprüht. Je nach Art des Klebstoffes wird durch Trocknung im Vakuum oder durch Vernetzung mittels UV-Licht getrocknet.

### Herstellungsbeispiel B3:

Die Beschichtung eines bereitgestellten handelsüblichen Angioplastie-Ballons erfolgt entsprechend einem der Herstellungsbeispiele B1 oder B2.

### C) Erfindungsgemäße Beschichtung eines Medizinprodukt mittels eines Tauchverfahrens:

### Herstellungsbeispiel C1:

Ein bereitgestellter Stent wird in eine stabilisierte Suspension umfassend Wirkstoffpartikel, vorzugsweise die gemäß Herstellverfahren A) hergestellten Nanopartikel, hydrophiles Polymer (Kohlenhydrate, Hyaluronsäure und/oder Gelatine), Netz- bzw. Dispergieradditiv sowie hochreines Wasser getaucht und anschließend im Vakuum getrocknet.

### Herstellungsbeispiel C2:

Die Beschichtung eines bereitgestellten handelsüblichen Angioplastie-Ballons erfolgt entsprechend Herstellungsbeispiel C1.

## Patentansprüche

1. Medizinprodukt implantierbar oder einführbar in ein Gefäßsystem eines menschlichen oder tierischen Organismus mit einer Wirkstoffbeschichtung auf der Oberfläche des Medizinproduktes, **dadurch gekennzeichnet, dass**
a) die Wirkstoffbeschichtung ein oder mehrere separate Wirkstoffpartikel umfassend ein oder mehrere Wirkstoffe und ein oder mehrere Polymere mit einem Partikeldurchmesser kleiner oder gleich 30 µm umfasst oder daraus besteht,
b) das oder die Wirkstoffpartikel unter physiologischen Bedingungen im Gefäßsystem aus der Beschichtung des Medizinproduktes ablösbar sind und
c) das oder die abgelösten Wirkstoffpartikel den oder die Wirkstoffe bei einem pH-Wert im Bereich von 7,3 bis 7,5 im Wesentlichen nicht freisetzen.

2. Medizinprodukt nach Anspruch 1, **dadurch gekennzeichnet, dass** das oder die Polymere des Wirkstoffpartikels ausgewählt werden aus pH-sensitiv degradierbaren Polymeren.

3. Medizinprodukt gemäß Anspruch 1 oder 2, **dadurch gekennzeichnet, dass** das pH-sensitiv degradierbare Polymer ausgewählt wird aus der Gruppe bestehend aus Poly(β-aminoestern), Poly(ethylenoxid) modifizierten Poly(β-aminoestern), Polylactiden, Poly(lactid-co-glycoliden), modifizierten Cyclodextrinen, modizifizierten Kohlenhydraten und/oder sonstigen Polyestern.

4. Medizinprodukt gemäß einem der vorstehenden Ansprüche, **dadurch gekennzeichnet, dass** die Wirkstoffpartikel auf der Oberfläche des Medizinproduktes (i) in eine degradierbare Polymerschicht inkorporiert oder (ii) mittels Haftmaterial auf der Oberfläche des Medizinproduktes als Wirkstoffbeschichtung vorliegen.

5. Medizinprodukt nach Anspruch 4, **dadurch gekennzeichnet, dass** (i) die degradierbare Matrix ausgewählt wird aus ein oder mehreren Materialien der Gruppe bestehend aus Polydioxanonen; Polycaprolactonen, Polyglycoliden; Polylactiden sowie Blends, Co-Polymere und Tri-Polymere hiervon; Polysacchariden; Polyhydroxyvaleraten; Ethylvinylacetaten; Polyethylenoxiden; Polyphosphorylcholinen; Fibrin; Albumin; und/oder Polyhydroxybuttersäuren sowie deren Blends.

6. Medizinprodukt nach Anspruch 4, **dadurch gekennzeichnet, dass** (ii) das Haftmaterial ausgewählt wird aus der Gruppe bestehend aus Kohlenhydraten; (Soja-)Lecithin; (Oligo-)Peptiden und/oder schnell degradierbaren Polyestern.

7. Medizinprodukt gemäß einem der vorstehenden Ansprüche, **dadurch gekennzeichnet, dass** die Wirkstoffe ausgewählt werden aus der Gruppe bestehend aus Antiphlogistika; Cytostatika; Immunsuppressiva; Thrombozytenaggregationshemmer; Statinen; Estrogenen; Lipidregulatoren; Vasodilatatoren; Calciumkanalblocker; Calcineurininhibitoren; Antünflammatorika; Antiallergika; Oligonucleotiden; Endothelbildner; Steroiden; Proteinen/Peptiden; Proliferationshemmer; Analgetika und Antirheumatika; Endothelinrezeptor-Antagonisten; Rho-Kinase Inhibitoren; RGD-Peptiden; und/oder organischen Goldverbindungen.

8. Medizinprodukt nach einem der vorstehenden Ansprüche, **dadurch gekennzeichnet, dass** das Medizinprodukt ein Wirkstoff-eluierender Stent ist.

9. Medizinprodukt nach Anspruch 8, **dadurch gekennzeichnet, dass** der Wirkstoff-eluierende Stent ein degradierbarer Metallstent ist.

10. Medizinprodukt nach Anspruch 8 oder 9, **dadurch gekennzeichnet, dass** die Wirkstoffbeschichtung auf der muralen Oberfläche des Stents aufgebracht ist.

11. Medizinprodukt nach einem der Ansprüche 1 bis 7, **dadurch gekennzeichnet, dass** das Medizinprodukt ein Wirkstoff-eluierender Angioplastie-Ballon ist.

12. Verfahren zur Herstellung eines in ein Gefäßsystem eines menschlichen oder tierischen Organismus implantierbaren oder einführbaren Medizinproduktes mit einer Wirkstoffbeschichtung, **dadurch gekennzeichnet, dass**
a) ein oder mehrere separate Wirkstoffpartikel bereitgestellt werden, die ein oder mehrere Wirkstoffe und ein oder mehrere Polymere umfassen und einen Partikeldurchmesser kleiner oder gleich 30 µm aufweisen,
b) ein Grundkörper eines Medizinproduktes bereitgestellt wird,
c) der Grundkörper aus Schritt b) mit den Wirkstoffpartikeln aus Schritt a) so beschichtet wird, dass die Wirkstoffpartikel im beschichteten Zustand unter physiologischen Bedingungen im Gefäßsystem aus der Beschichtung des Medizinproduktes ablösbar sind, aber der oder die Wirkstoffe bei einem pH-Wert im Bereich von 7,3 bis 7,5 im Wesentlichen nicht aus den abgelösten Wirkstoffpartikeln freigesetzt werden.

13. Wirkstoffbeschichtung für ein in ein Gefäßsystem eines menschlichen oder tierischen Organismus implantierbares oder einführbares Medizinprodukt, **dadurch gekennzeichnet, dass**
a) die Wirkstoffbeschichtung ein oder mehrere separate Wirkstoffpartikel umfassend ein oder mehrere Wirkstoffe und ein oder mehrere Polymere mit einem Partikeldurchmesser kleiner oder gleich 30 µm umfasst oder daraus besteht,
b) das oder die Wirkstoffpartikel unter physiologischen Bedingungen im Gefäßsystem aus der Beschichtung des Medizinproduktes ablösbar sind und
c) das oder die abgelösten Wirkstoffpartikel den oder die Wirkstoffe bei einem pH-Wert im Bereich von 7,3 bis 7,5 im Wesentlichen nicht freisetzen.

14. Verwendung der Wirkstoffbeschichtung nach Anspruch 13 zur Herstellung eines in ein Gefäßsystem eines menschlichen oder tierischen Organismus implantierbaren oder einführbaren Medizinprodukts.

15. Verfahren zur Behandlung einer Stenose, einer calcifizierten oder weichen Plaque oder eines Aneurysma im Gefäßsystem eines menschlichen oder tierischen Organismus, **dadurch gekennzeichnet, dass** ein wirkstoffbeschichtetes Medizinprodukt nach einem der Ansprüche 1 bis 11 bereitgestellt wird und in das Gefäßsystem des menschlichen oder tierischen Organismus implantiert oder eingeführt wird.
